# EUROPEAN PATENT SPECIFICATION

(11) **EP 3 469 124 B1**
(45) Date of publication and mention of the grant of the patent: **05.08.2020**
(21) Application number: 17734561.8
(22) Date of filing: 12.06.2017
(51) Int. Cl.: D01F 1/10, G01N 33/26, D02G 3/04

(54) **METHOD FOR COMMUNICATING FIBER PROPERTIES OF A YARN**
VERFAHREN ZUR KOMMUNIKATION DER FASEREIGENSCHAFTEN EINES GARNS
PROCÉDÉ PERMETTANT DE COMMUNIQUER DES PROPRIÉTÉS DE FIBRE D'UN FIL

(30) Priority: 13.06.2016 US 201662349286 P
(43) Date of publication of application: 17.04.2019
(73) Proprietor: INVISTA Textiles (U.K.) Limited, Manchester M2 3DE (GB)
(72) Inventor: GULLEDGE, Alexander L., Columbia South Carolina 29229 (US); AGARWAL, Nirmal Kumar, Columbia South Carolina 29229 (US)
(74) Representative: Michalski Hüttermann & Partner Patentanwälte mbB
(86) International application number: PCT/US2017/036993
(87) International publication number: WO 2017/218401

(56) References cited:
- WO-A1-97/32062
- AU-A- 3 565 071
- US-A1- 2003 198 809
- US-A1- 2010 063 208

## Description

This patent application claims the benefit of priority from U.S. Provisional Application Serial No. 62/349,286, filed June 13, 2016.

### Field of Invention

The present disclosure relates to methods for communicating one or more fiber properties in a yarn and articles comprising a yarn which arc capable of communicating one or more of their fiber properties upon analysis. The present disclosure also relates to methods for intensifying color of a yarn and articles comprising yarn with the intensified color. The present disclosure also relates to improvements in bulked continuous filament fiber processing through substitution of problematic colorants.

### Background

Product tags are used throughout the textile industry to label, identify, or otherwise mark a product.

Finish tints, which are colored oils or liquid dispersions, are frequently used for bulked continuous filament (BCF) fiber spinning applications, as a way to identify or tag the fiber bundle to designated fiber properties. For example, since nylon fibers are produced with e.g. different dyeing, anti-static, and viscosity properties, the application of tint is a necessity for carpet mills when tufting constructions of various nylon BCF fiber types, e.g. white-dyeable nylon fibers and yarns. Tinted fibers enable textile and carpet mills to e.g. verify correct creel placement when producing multi-depth constructions, and track incorporation of one or more particular fiber types into the finished good. An advantage of tints for the BCF fiber producer is e.g. the ability to monitor product placement and usage at points downstream of fiber spinning processes.

A major downside to use of tints in industrial settings is that they present a number of interrelated production issues. For example, during high-speed BCF fiber production, tint can be difficult to contain as the tinted fiber passes through the tufting process. Finish tint creates a residue on equipment contact points, and creates unwanted splatter patterns in production areas. As such, production runs arc impacted as there is time spent cleaning tint residue off of equipment. Changing tinted products requires time consuming cleanups. Tinted yarns will also "bleed," and so the tint will contaminate other fibers or yarns upon contact.

As alternative product tags, U.S. Patent 5,217,646 discloses a fabric coating fluid comprising a mixture of an ultraviolet light indicating dye and a fabric protecting ingredient in an evaporative carrier for coating on a fabric to provide, after evaporation, a coating invisible to ambient light but visible when exposed to ultra-violet light to indicate the presence of the protective coating.

Published U.S. Patent Application No. 2003/0198809 discloses a fluorescent elastic yarn which can be discriminated by fluorescence when ultraviolet light is irradiated to it.

US RE42,188 E discloses fibers suggested to possess unique and difficulty duplicated combinations of complex cross-sections, components and multiple luminescent responses which protect against fraudulent duplication of security articles comprised of these fibers.

Optical brighteners are commonly used in laundry detergents for their natural whitening ability (Jasperse ct al. Journal of the American Oil Chemists Society 1992 69(7);621-625). Optical brighteners work on a principle known as a Stokes Shift (Gispert, J. R. 2008 Coordination Chemistry Wiley-VCH. P 483). This is a phenomenon in which a photon is absorbed and re-emitted at a longer wavelength of light. The difference in wavelength between the absorbed and emitted photon is known as the Stokes Shift (Gispert, J. R. 2008 Coordination Chemistry Wiley-VCH. P 483). In the case of optical brighteners, absorption occurs at the edge of the UV wavelength region and emission occurs at the beginning of the visible spectrum. This produces a "whitening" effect as the relatively short wavelength blue light has a visually whiter appearance.

U.S. Patent 6,150,494 discloses water-dispersible polymeric compositions containing optical brightener compounds copolymerized therein useful in formulation of optical brightener inks, paints and film forming compositions. The optical brighteners must possess at least one polyester reactive group which can be copolymerized with a dicarboxylic acid component, diol and/or diamine component and a sulfonate containing monomer to produce the polymer.

EP 1 709 220 B1 discloses synthetic polyamide compositions with improved whiteness by incorporating an optical brightener into the yarn itself. These polyamide compositions also contain an anti-oxidant stabilizer and optionally an antimicrobial additive. The optical brightener is present in an amount of about 0.005 to about 0.2 percent by weight of the composition.

Published U.S. Patent Application No. 2008/0090945 discloses a polyamide composition inclusive of an optical brightener together with an anti-oxidant stabilizer that exhibits superior whiteness after heat setting.

U.S. Patent 7,338,877 discloses a multicomponent fiber having a non-luminescent first polymeric component and less than 50 percent of a second polymeric component comprising a luminescent component. These fibers are suggested to be useful in the manufacture of safety apparel and equipment.

Published U.S. Patent Application No. 2013/0008621 discloses a monofilament yarn for a paper machine clothing fabric with a UV additive such as an optical brightener or fluorescent whitening agent that makes the yarn ends easily visible using a light or radiation source.

U.S. Patent 8,408,766 discloses a surface material which emits radiation in response to exposure to a given light source. Such surface materials are comprised of fibers treated with an optical brightener by spraying or dyeing or mixed with luminous particles and are suggested to be useful as a vehicular trim component to improve illumination.

### Summary of the Invention

An aspect of the present invention relates to a method of communicating one or more fiber properties in a multifilament yarn as defined in claim 1. Fluorescent fibers are *inter alia* known from AU 35650 71 A, US 2010/063208 A1, and WO 97/32062 A1.

In one nonlimiting embodiment, the fiber forming polymer of the yarn is a polyamide, a polyester, a polyolefin, a poly(*para*-aramid), a polyimide or a blend or copolymer thereof.

In one nonlimiting embodiment, the fiber forming polymer is a polyamide such as nylon 6, nylon 6,6, or a blend or copolymer thereof.

In one nonlimiting embodiment, the fiber forming polymer is a polyester such as poly(ethylene terephthalate), poly(ptopylene terephthalate), poly(butylene terephthalate) or a blend or copolymer thereof.

In the present invention, the fiber in the multifilament yarn or multifilament yarn bundle further comprising the fiber property identification additive comprises from about 0.1 to about 4 weight percent of the fiber in the yarn.

In one nonlimiting embodiment, the fiber property identification additive present in the fiber of the multifilament yarn or multifilament yarn bundle is not present in the backbone, or main chain, of the fiber forming polymer.

In one nonlimiting embodiment, the fiber property identification additive is an optical brightener, a fluorescent whitening agent, an inorganic taggant, or any combination thereof.

In one nonlimiting embodiment, the fiber property identification additive is an optical brightener such as a stilbene, a coumarin compound, a carbostyril compound, a diphenylpyrazoline, a naphthamide, a benzoxazolyl compound, or any combination thereof.

In one nonlimiting embodiment, the fiber in the multifilament yarn or multifilament yarn bundle further comprising the fiber property identification additive docs not comprise a tint additive.

In one nonlimiting embodiment, analyzing the multifilament yarn or multifilament yarn bundle to identify one or more fiber properties does not require a surface treatment on the fibers.

In one nonlimiting embodiment, the fiber property communicated is a property or characteristic such as anti-static, stain resistant, soil repellant, cationic dycable, core-shell bicomponent, relative dyeability factor, relative viscosity range, anti-microbial, anti-bacterial, polymer composition, anti-allergen, delustered, or any combination thereof.

In one nonlimiting embodiment, the concentration of the fiber property identification additive present in the fiber is indicative of the fiber property.

Another aspect of the present invention relates to a multifilament yarn or multifilament yarn bundle which communicates one or more fiber properties in the multifilament yarn or multifilament yarn bundle by addition of a portion of fiber comprising a fiber property identification additive to the multifilament yarn or multifilament yarn bundle.

In one nonlimiting embodiment, the fiber property identification additive present in the fiber is in range from about 50 to about 4000 ppm.

In one nonlimiting embodiment, the fiber forming polymer of the multifilament yarn or multifilament yarn bundle is a polyamide, a polyester, a polyolefin, a poly(*para*-aramid), a polyimide or a blend or copolymer thereof.

In one nonlimiting embodiment, the fiber forming polymer is a polyamide such as nylon 6, nylon 6,6, or a blend or copolymer thereof.

In one nonlimiting embodiment, the fiber forming polymer is a polyester such as poly(ethylene terephthalate), poly(propylene terephthalate), poly(butylene terephthalate) or a blend or copolymer thereof.

In the present invention, the fiber in the multifilament yarn or multifilament yarn bundle further comprising the fiber property identification additive comprises from about 0.1 to about 4 weight percent of the fiber in the multifilament yarn or multifilament yarn bundle.

In one nonlimiting embodiment, the fiber property identification additive present in the fiber of the multifilament, yarn or multifilament yarn bundle is not present in the backbone of the fiber forming polymer.

In one nonlimiting embodiment, the fiber property identification additive is an optical brightener, a fluorescent whitening agent, an inorganic taggant or any combination thereof.

In one nonlimiting embodiment, the fiber property identification additive is an optical brightener such as a stilbene, a coumarin compound, a carbostyril compound, a diphenylpyrazoline, a naphthamide, a benzoxazolyl compound or any combination thereof.

In one nonlimiting embodiment, the fiber in the multifilament yarn or multifilament yarn bundle further comprising the fiber property identification additive does not comprise a tint additive.

In one nonlimiting embodiment, analyzing the multifilament yarn or multifilament yarn bundle to identify one or more fiber properties does not require a surface treatment on the fibers.

In one nonlimiting embodiment, the fiber property communicated is a property or characteristic such as anti-static, stain resistant, soil repellant, cationic dyeable, core-shell bicomponent, relative dyeability factor, relative viscosity range, anti-microbial, anti-bacterial, polymer composition, anti-allergen, or delustered or any combination thereof.

In one nonlimiting embodiment, concentration of the fiber property identification additive present in the fiber of the multifilament yarn or multifilament yarn bundle is indicative of the fiber property.

Another aspect of the present invention relates to a method of intensifying color of a multifilament yarn or multifilament yarn bundle by addition of an optical brightener.

In one nonlimiting embodiment, the color intensified is a dark color.

In one nonlimiting embodiment, the color intensified is black.

In one nonlimiting embodiment, the optical brightener used to intensify the color is a stilbene, a coumarin compound, a carbostyril compound, a diphenylpyrazoline, a naphthamide, a benzoxazolyl compounds or any combination thereof.

Yet another aspect of the present invention relates to improvements in bulked continuous filament fiber processing through substitution of problematic colorants with a fiber property identification additive.

### Brief Description of the Figures

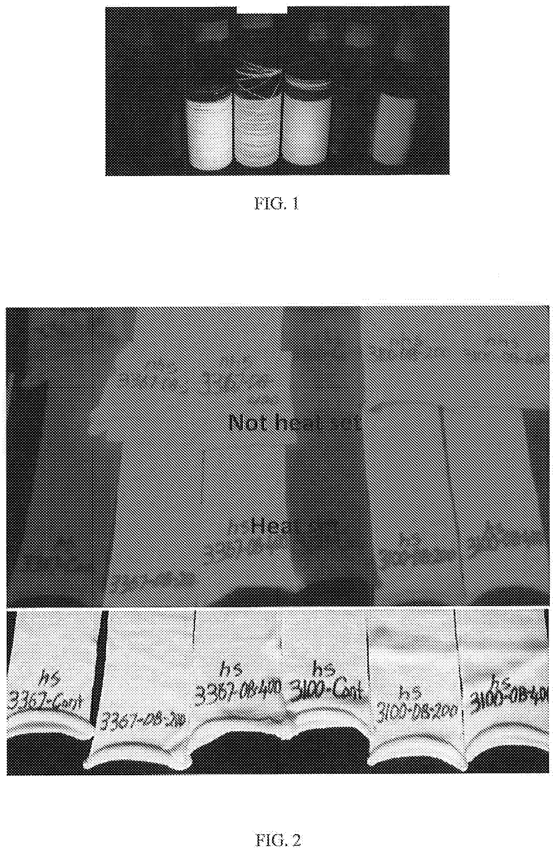

FIG. 1 is an image showing color variation with concentration with respect to control in multifilament yarn bundles with various concentrations of optical brightener as the fiber property identification additive. Additive concentrations as shown from left to right are: 0 ppm, 10,000ppm, 2500 ppm, 500 ppm, 0 ppm, 250 ppm.
FIG. 2 arc photographs of knit socks made from white dyeable and sulfonated N66 yarns. From top to bottom, under UV exposure non-heatset and Superba® heat set knit socks followed by the Superba® heat set socks under visible light. From left to right, white N66 control, 200ppm optical brighten, 400ppm optical brightener, sulfonated N66 control, sulfonated N66 with 200 and then 400 ppm optical brightener additive.

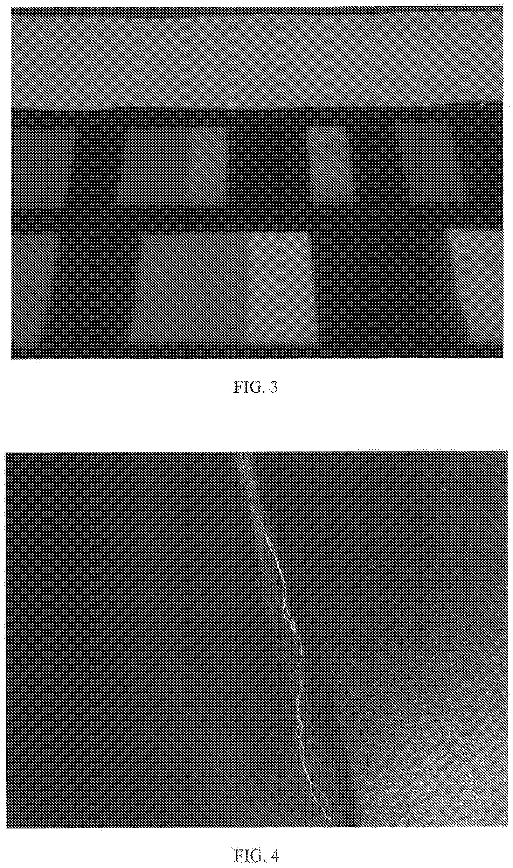

FIG. 3 is a photograph of knit socks under ambient lighting (top) and under UV lighting (middle). The bottom image is a closer look at the same knit sock under a UV light source. The bands represent 0, 200, and 400 ppm of the optical brightener additive, with the brightness of the bands corresponding to increasing levels of the additive.
FIG. 4 is an image of a multifilament yarn of the present invention.

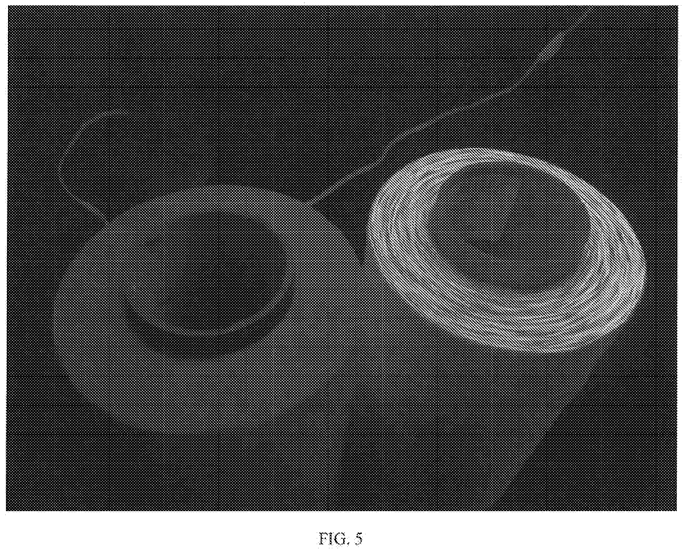

FIG. 5 is an image of spools of a blue pigmented yarn without (left) and with (right) a fiber property identification additive under UV illumination.

### Detailed Description of the Invention

Provided by this disclosure are methods of communicating one or more fiber properties in a multifilament yarn or multifilament yarn bundle and yarns, yarn bundles and articles comprising such yarns capable of communicating one or properties of the fibers therein.

In the methods, yarns and yarn bundles of the present invention, a multifilament yarn comprising at least one fiber formed from a fiber forming polymer and further comprising a fiber property identification additive which can be analyzed to identify one or more fiber properties is provided.

Examples of fiber forming polymers of the multifilament yarn and multifilament yarn bundles include, but arc not limited to polyamides, polyesters, polyolefins, poly(*para*-aramids), polyimides and blends or copolymers thereof. Examples of polyamides useful as the fiber forming polymers include, but are not limited to, nylon 6, nylon 6,6, and blends or copolymers thereof. Examples of polyesters useful as fiber forming polymers include, but are not limited to poly(ethylene terephthalate), poly(propylene terephthalate), poly(butylene terephthalate) and blends or copolymers thereof.

Examples of fiber property identification additives of the multifilament yarn and multifilament yarn bundles include, but are not limited to, optical brighteners, fluorescent whitening agents, inorganic taggants and any combinations thereof. Examples of optical brighteners useful as fiber property identification additives include, but are not limited to, stilbenes, coumarin compounds, carbostyril compounds, diphenylpyrazolines, naphthamides, benzoxazolyl compounds and any combinations thereof. In one nonlimiting embodiment, the fiber property identification additive is 4,4'-bis(2-benzoxazolyl)stilbene. In another nonlimiting embodiment, the fiber property identification additive is 2,5-thiophenediylbis (5-*t*-butyl-1,3-benzoxazole).

The fiber property identification additive is present in the fiber in range from about 10 to about 4000 ppm.

In one nonlimiting embodiment, the fiber property identification additive present in the fiber of the multifilament yarn or multifilament yarn bundle is not present in the backbone of the fiber forming polymer.

With the incorporation of a fiber property identification additive in accordance with the present invention, the currently used practice of tint for product identification may be reduced or eliminated. Accordingly, in one nonlimiting embodiment, the fiber in the multifilament yarn or multifilament yarn bundle further comprising the fiber property identification additive does not comprise a tint additive.

The method of the present invention further comprises analyzing the multifilament yarn or multifilament yarn bundle to identify one or more fiber properties. In one nonlimiting embodiment, analyzing the yarn or yarn bundle to identify one or more fiber properties does not require a surface treatment on the fibers. In one nonlimiting embodiment, properties of the fibers of the yarn or yarn bundle may be identified by UV irradiation at a selected wavelength.

Fiber in the multifilament yarn or multifilament yarn bundle further comprising the fiber property identification additive comprise from 0.1 to 4 weight percent of the fiber in the multifilament yarn or multifilament yarn bundle.

Examples of fiber properties of the multifilament yarn or multifilament yarn bundle communicated by the fiber property identification additive include, but are not limited to, anti-static, stain resistant, soil repellant, cationic dyeable, core-shell bicompouent. relative dyeability factor, relative viscosity range, anti-microbial. anti-bacterial, polymer composition, anti-allergen. and delustered as well as any combinations thereof

Typical industrial BCF fiber production uses multiple tint colorations. Examples of tint colorations are: blue, green, purple, red, and yellow. Therefore, for a fiber property identification additive such as an optical brightener to replace tint as an identifying agent, it must be able to produce distinct signals, such as from several variations of visible color. Variation of color can be achieved in a few ways. For example, in one embodiment, color variation can be achieved through concentration variation. By varying the concentration of additive used, one can vary the resulting color. This effect is demonstrated with an optical brightener as the fiber property identification additive in FIGs. 1-5. In practice, one might use no fiber property identification additive in the highest volume product so that no fluorescence indicates its identity. The next highest volume white product could use the lowest possible amount of fiber property identification additive which would tag it while continuing to minimize cost. Several tints arc thus replaced through the use of predetermined fiber ultraviolet light absorption response, as the manner for communicating the fiber property of interest. Accordingly, in one nonlimiting embodiment, the concentration of the fiber property identification additive present in the fiber is indicative of the fiber property.

Examples of optical brighteners which can be used in this method include, but arc not limited to stilbenes, coumarin compounds, carbostyril compounds, diphenylpyrazolines, naphthamides, benzoxazolyl compounds and any combination thereof. In an alternative nonlimiting embodiment, a chemical derivative that still functions as an optical brightener but with a shifted wavelength emission could be used.

Also provided by the present invention are articles comprising yarn capable of communicating one or properties of the fibers therein. Examples of such articles include any textile in which bulked continuous fiber is used. In one nonlimiting embodiment, the article is carpet. In another nonlimiting embodiment, the article is a garment.

The present disclosure also relates to methods for intensifying color of multifilament yarn or multifilament yarn bundles and articles comprising yarn with the intensified color. In these methods, an optical brightener is added to the multifilament yarn.

Nonlimiting examples of fiber forming polymers of the multifilament yarn and multifilament yarn bundles with intensified color include, but are not limited to polyamides, polyesters, polyolefins, poly(*para*-aramids), polyimides and blends or copolymers thereof. In one nonlimiting embodiment, the fiber forming polymer is a polyamide such as nylon 6, nylon 6,6, or a blend or copolymer thereof. In one nonlimiting embodiment, the fiber forming polymer is a polyester such as poly(ethylene terephthalate), poly(propylene terephthalate), poly(butylene terephthalate) or a blend or copolymer thereof.

Nonlimiting examples of optical brighteners which can be used in this method include, but are not limited to stilbenes, coumarin compounds, carbostyril compounds, diphenylpyrazolines, naphthamides, benzoxazolyl compounds and any combination thereof.

Various colors can be intensified. In one nonlimiting embodiment, the color intensified is a dark color. In one nonlimiting embodiment, the color intensified is black.

The effects of optical brighteners as fiber property identification additives in white-dyeable and solution-dyed Nylon 6,6 fibers to replace conventional product tagging methods and achieve higher process yield were evaluated. In these experiments, an organic optical brightener was incorporated in nylon 6,6 processing via melt addition. The additive can also be added into the autoclave reactor, in combination with monomeric or oligomeric ingredients, via liquid injection processes as are well-understood in the industry. The organic optical brightener used in these experiments was 4 4'-bis(benzoxazolyl)-cis-stilbene. See Formula I below:

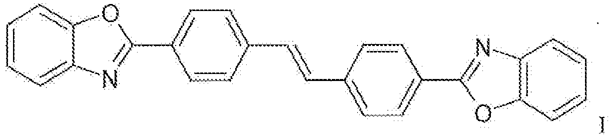

However, as will be understood by the skilled artisan upon reading this disclosure, alternative optical brighteners including, but not limited to, other stilbenes. coumarin compounds, carbostyril compounds. diphenylpyrazolines, naphthamides, benzoxazolyl compounds and any combinations thereof can be used as well as fluorescent whitening agents, inorganic taggants and any combinations thereof. Additional alternative nonlimiting examples of commercially available optical brighteners useful in the present invention include Formulas II and III: 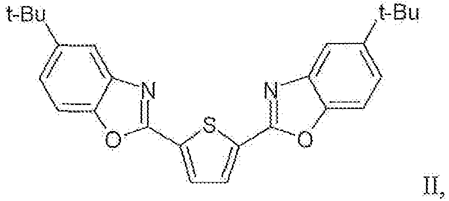 and

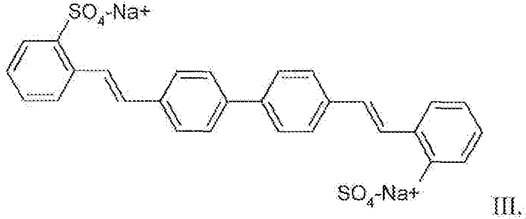

The optical brightener master-batch used was incorporated in nylon 6,6 BCF spinning by melt addition at the extruder intake. No significant processing issues were observed by this addition. The optical brightener was evaluated at 200 ppm and 400 ppm active ingredient loading levels with solution dyed nylon (SDN) and white dyeable nylon. SDN filament material was produced using a twin screw extruder, in an array of 26 single pigment colors, while white dyeable products were produced using a single screw extruder asset

In addition, varying loadings of a single optical brightener additive were added in accordance with the present invention to demonstrate utility in identification of different fiber properties via variation of concentration of the fiber identification additive. Results arc shown in FIGs. 1 through 3.

As shown in FIG. 1, by altering the concentration of the fiber property identification additive, the emission level of light from the additive can he adjusted, thus changing the overall color perceived. A single excitation wavelength of 490 nm was used in these experiments. FIG. 1 shows how the color varies with changes in concentration of the additive, with respect to the control (0 ppm additive) sample. It is expected that by incorporating even lower levels of additive, a more purple/violet color can be achieved. The knit socks in FIG. 2 demonstrate the replacement of three tints using the brightness tags "OFF", "Low" (200ppm), and "Bright" (400ppm). As shown in FIG. 2, low levels of the optical brightener additive (200 and 400 ppm) showed sufficient brightness in white yarns to allow for the distinction between those yarns under UV wavelength excitation. Further, pigmented yarns with these loadings were bright enough under UV to provide an aesthetic accent that may be appealing in certain end-uses. An image of a sock made from blue pigmented yarns with 0, 200, and 400 ppm additive after heat setting using a SUPERBA machine (American Superba, Inc., Dalton, GA USA) is shown in FIG. 3 under visible and UV illumination for comparison. No difference in the fluorescence was observable to the naked eye following this heat setting treatment.

Mechanical properties of sulfonated N66 polymer made with and without the additive on a single screw extrusion fiber spinning asset were measured, and the results are shown in Table 4. Modulus was slightly reduced and mean elongation slightly increased upon the addition of 200 and 400 ppm of the additive, while yarn tenacity was essentially unchanged. However, the presence of increasing amounts of additive yielded a fiber with better retention of tenacity and modulus after exposure to 160 AFU (Acelerated fading Unit).

**Table 4: Table of results from tensile testing of 1245 denier yarns made with sulfonated N66 polymer**

| | **3100 - Control** | | | **3100 - OB - 200** | | | **3100 - OB - 400** | |
|---|---|---|---|---|---|---|---|---|
| | Unexposed | 160 AFU Exposed | | Unexposed | 160 AFU Exposed | | Unexposed | 160 AFU Exposed |
| **Mean Elongation (%)** | 46.04 | 19.54 | | 54.39 | 27.61 | | 56.57 | 35.76 |
| Std.Dev. | 3.17 | 0.93 | | 3.02 | 1.52 | | 4.59 | 3.33 |
| **Reduction in Mean Elongation after 160 AFU** | -- | 58% | | -- | 49% | | -- | 37% |
| **Tenacity (gf/den)** | 3.10 | 1.80 | | 3.24 | 2.16 | | 2.97 | 2.17 |
| **Tenacity Retention after 160 AFU** | -- | 58% | | -- | 67% | | -- | 73% |
| Std.Dev. | 0.09 | 0.03 | | 0.07 | 0.06 | | 0.08 | 0.08 |
| **Energy at Maximum Load (lbf)** | 8.50 | 4.93 | | 8.91 | 5.93 | | 8.16 | 5.96 |
| Std.Dev. | 0.24 | 0.08 | | 0.20 | 0.18 | | 0.23 | 0.22 |
| **Modulus (gf/den)** | 14.31 | 12.43 | | 13.10 | 11.44 | | 13.31 | 12.11 |
| Std.Dev. | 0.21 | 0.57 | | 0.68 | 0.76 | | 0.76 | 0,55 |
| **Reduction in Modulus after 160 AFU** | -- | 13% | | -- | 13% | | -- | 9% |

The addition of a fiber property identification additive that can be analyzed to identify one or more fiber properties to a fiber forming polymer in accordance with the present invention was compatible at all processing conditions. Further, variation in concentration of the fiber property identification additive was demonstrated to produce detectable differences useful in product tagging. Accordingly, utilization of these fiber property identification additives in BCF production could provide direct process improvements to reduce time and material losses that currently occur with conventional product tagging methods involving tint.

Communicating anti-stat characteristic in a synthetic fiber is also achieved according to the present disclosure. In one instance, anti-stat fiber is inserted with standard fiber during BCF production and is used to mitigate electrostatic build-up on fibers. In order to achieve this effect, the anti-stat fiber must measure near zero conductivity. Anti-static fiber is, in one nonlimiting example, a bicomponent filament, composed of an electronically insulating core, and an electronically conducting sheath. The use of anti-stat fiber in BCF spinning operations outside of predetermined use rates can produce color streaks when these products are tufted with yarn types not containing the same amount of anti-stat additive. Since the anti-stat is virtually invisible to the naked eye, incorporation of a fiber property identification additive in accordance with the present disclosure can greatly improve anti-stat detection. In addition to this, the presence of a fiber property identification additive allows for quick quality assurance checks and prevents misplacement of the fiber during creel string up, and BCF insertion.

When used in conjunction with UV based emergency lighting, articles such as, but not limited to, carpeting prepared from the yarn comprised of the present disclosure also find use as a built-in safety feature. As a nonlimiting example, the yarn can be incorporated into the carpeting, so as to form a path or arrows leading or pointing to a safe exit when UV based emergency lighting is activated.

Further, as a result of the incorporation of a fiber property identification additive, it was surprising found that more than two times the amount of problematic blue pigment colorants could be removed from a fiber with the replacement of the active component introduced herein. Moreover, removal of the problematic blue pigments used in standard processing allowed for a process improvement in yield and processability. Accordingly, the method of the present invention also provides for improved bulked continuous filament fiber processing through substitution of problematic colorants with a fiber property identification additive such as, but not limited to, an optical brightener.

The following section provides further illustration of the methods and yarn bundles of the present invention. These working examples arc illustrative only and are not intended to limit the scope of the invention in any way.

### EXAMPLES

### Example 1: Yarn Production (Reference)

The optical brightener additive used in these experiments was obtained from a masterbatch supplier as OB Natural. The additive came as a 5% loading in nylon 6,6 and had a neon greenish tint. The additive was used as received without further processing or incident.

The optical brightener masterbatch used was incorporated in nylon 6,6 BCF spinning through melt addition at the extruder. While injection of the additive at the extruder presented a drop in melt viscosity, it was not significant enough to cause processing issues. The extrusion temperature used for nylon 6,6 BCF fiber spinning was around 280°C. For these experiments, the optical brightener was evaluated at 200 ppm and 400 ppm active ingredient loading levels. Trials with solution dyed nylon (SDN) and white dyeable nylon were conducted. SDN material was produced on a pilot spinning asset in an array of 26 single pigment colors, while white dyeable products were produced on a single extruder spinning asset. The yarns made from this example are depicted in FIG. 1.

### Example 2: Knitted Socks (Reference)

Socks were knit for probing pertinent performance parameters. A portion of each of sock was also SUPERBA heatset at 265°F (129.4°C), to ensure that the more open polymer structure that results from heat-setting is not further compromised by the new polymer compositions.

### Example 3: Multicomponent Fiber

Two filament, multicomponent fibers were produced using the optical brightener described herein as a component of the sheath with a conductive core component. These fibers showed ease of detectability and did not impair standard fiber properties of tenacity, elongation, conductivity, or denier. These multicomponent fibers were added to yarn via insertion into the thread-line bundle during processing to impart the active characteristics described. The resulting processed yarn then embodied the detectability and property communication attributes of the inserted multicomponent fiber. This yarn was then processed into articles such as carpet and again the effect of detectability and communication of fiber properties was maintained.

### Example 4: Yield improvement from Substitution of Problematic Colorants (Reference)

The optical brightener described herein was added to solution dyed fiber containing blue pigments known to cause problems during Nylon 6,6 fiber spinning and process yield losses. As a result of the incorporation, it was surprising that more than two times the amount of blue pigment colorants could be removed from the fiber with the replacement of the active component introduced herein. Moreover, removal of the problematic blue pigments used in standard processing allows for a process improvement in yield and processability.

### Example 5: Yarn Identification

Multifilament fiber samples were produced of varying dyability polymer types (Cat-dye [5,000ppm], light-dye [50ppm], regular-dye [500ppm], deep-dye [50,000ppm]). The optical brightener described herein was added at unique concentrations for each of the sample types. The unique concentrations used were sufficient enough to distinguish between the different polymer type samples and communicate dyability of the fibers produced by irradiation with UV light under ambient lighting conditions.

## Claims

1. A method of communicating one or more fiber properties in a multifilament yarn, comprising the steps of:
a) providing a multifilament yarn comprising at least one fiber formed from a fiber forming polymer and comprising a fiber property identification additive present in the fiber in a range from 10 to 4000 ppm, wherein the fiber property identification additive can be analyzed to identify one or more fiber properties; and
b) analyzing the multifilament yarn to identify one or more fiber properties;
wherein the fiber from step (a) comprises from 0.1 to 4 weight percent of the multifilament yarn.

2. The method of claim 1, wherein the fiber forming polymer is selected from the group consisting of a polyamide, polyester, polyolefin, poly(*para*-aramid), and polyimide and blends and copolymers thereof.

3. The method of claim 1, wherein analyzing the multifilament yarn to identify one or more fiber properties does not require a surface treatment on the fiber from step (a).

4. The method of claim 1, wherein the fiber property identification additive present in the fiber from step (a) is not present in the backbone of the fiber forming polymer.

5. The method of claim 1, wherein the fiber from step (a) does not comprise a tint additive.

6. The method of claim 1, wherein the one or more fiber properties are selected from the group consisting of anti-static, stain resistant, soil repellant, cationic dyeable, core-shell bicomponent, relative dyeability factor, relative viscosity range, anti-microbial, anti-bacterial, polymer composition, anti-allergen and delustered and combinations thereof.

7. The method of claim 1, wherein the fiber property identification additive is selected from the group consisting of an optical brightener, a fluorescent whitening agent, and an inorganic taggant and combinations thereof.

8. The method of claim 7, wherein the optical brightener is selected from the group consisting of stilbenes, coumarin compounds, carbostyril compounds, diphenylpyrazolines, naphthamides, and benzoxazolyl compounds and combinations thereof.

9. The method of claim 8 wherein the optical brightener is 4,4'-bis(2-benzoxazolyl)stilbene or 2,5-thiophenediylbis (5-*t*-butyl-1,3-benzoxazole).

10. A multifilament yarn which communicates one or more of its fiber properties, said multifilament yarn comprising a portion of fiber comprising a fiber forming polymer and a fiber property identification additive, wherein the fiber property identification additive present in the fiber is in range from 10 to 4000 ppm, and wherein the portion of fiber in the multifilament yarn comprising the fiber property identification additive comprises from 0.1 to 4 weight percent of the fiber in the multifilament yarn.

11. The multifilament yarn of claim 10 wherein the fiber forming polymer of the multifilament yarn is selected from the group consisting of a polyamide, a polyester, a polyolefin, a poly(*para*-aramid), and a polyimide and a blend or copolymer thereof.

12. The multifilament yarn of claim 10 wherein the fiber property identification additive is selected from the group consisting of an optical brightener, a fluorescent whitening agent, and an inorganic taggant and any combination thereof.

13. The multifilament yarn of claim 12 wherein fiber property identification additive is an optical brightener selected from the group consisting of stilbenes, coumarin compounds, carbostyril compounds, diphenylpyrazolines, naphthamides and benzoxazolyl compounds and combinations thereof.

14. The multifilament yarn of claim 13 wherein the fiber property identification additive is 4,4'-bis(2-benzoxazolyl)stilbene or 2,5-thiophenediylbis (5-*t*-butyl-1,3-benzoxazole).

15. The multifilament yarn of claim 10 wherein the fiber property communicated is a property selected from the group consisting of anti-static, stain resistant, soil repellant, cationic dyeable, core-shell bicomponent, relative dyeability factor, relative viscosity range, anti-microbial, anti-bacterial, polymer composition, anti-allergen, and delustered and any combination thereof.

## Patentansprüche

1. Verfahren zum Kommunizieren einer oder mehrerer Fasereigenschaften in einem Multifilamentgarn, umfassend die Schritte:
a) Bereitstellen eines Multifilamentgarns, das mindestens eine Faser umfasst, die aus einem faserbildenden Polymer gebildet ist und ein Fasereigenschaftsidentifikationsadditiv umfasst, das in der Faser in einem Bereich von 10 bis 4000 ppm vorhanden ist, wobei das Fasereigenschaftsidentifikationsadditiv analysiert werden kann, um eine oder mehrere Fasereigenschaften zu identifizieren; und
b) Analysieren des Multifilamentgarns, um eine oder mehrere Fasereigenschaften zu identifizieren;
wobei die Faser aus Schritt (a) zu von 0,1 bis 4 Gew.-% das Multifilamentgarn umfasst.

2. Verfahren nach Anspruch 1, wobei das faserbildende Polymer ausgewählt ist aus der Gruppe bestehend aus einem Polyamid, Polyester, Polyolefin, Poly(para-aramid) und Polyimid und Mischungen und Copolymeren davon.

3. Verfahren nach Anspruch 1, wobei das Analysieren des Multifilamentgarns, um eine oder mehrere Fasereigenschaften zu identifizieren, keine Oberflächenbehandlung an der Faser aus Schritt (a) erfordert.

4. Verfahren nach Anspruch 1, wobei das Fasereigenschaftsidentifikationsadditiv, das in der Faser aus Schritt(a) vorhanden ist, nicht im Rückgrat des faserbildenden Polymers vorhanden ist.

5. Verfahren nach Anspruch 1, wobei die Faser aus Schritt (a) kein Färbungsadditiv umfasst.

6. Verfahren nach Anspruch 1, wobei die eine oder mehreren Fasereigenschaften ausgewählt sind aus der Gruppe bestehend aus antistatisch, fleckenbeständig, schmutzabweisend, kationisch färbbar, Kern-Schale-Bikomponente, relativem Färbbarkeitsfaktor, relativem Viskositätsbereich, antimikrobiell, antibakteriell, Polymerzusammensetzung, antiallergen und mattiert und Kombinationen davon.

7. Verfahren nach Anspruch 1, wobei das Fasereigenschaftsidentifikationsadditiv ausgewählt ist aus der Gruppe bestehend aus einem optischen Aufheller, einem fluoreszierenden Weißmacher und einem anorganischen Taggant und Kombinationen davon.

8. Verfahren nach Anspruch 7, wobei der optische Aufheller aus der Gruppe bestehend aus Stilbenen, Cumarin-Verbindungen, Carbostyril-Verbindungen, Diphenylpyrazolinen, Naphthamiden und Benzoxazolyl-Verbindungen und Kombinationen davon ausgewählt ist.

9. Verfahren nach Anspruch 8, wobei der optische Aufheller 4,4'-Bis(2-benzoxazolyl)stilben oder 2,5-Thiophendiylbis(5-*t*-butyl-1,3-benzoxazol) ist.

10. Multifilamentgarn, das eine oder mehrere seiner Fasereigenschaften kommuniziert, wobei das Multifilamentgarn einen Faserabschnitt umfasst, der ein faserbildendes Polymer und ein Fasereigenschaftsidentifizierungsadditiv umfasst, wobei das Fasereigenschaftsidentifizierungsadditiv, das in der Faser vorhanden ist, im Bereich von 10 bis 4000 ppm liegt und wobei der Faserabschnitt in dem Multifilamentgarn, das das Fasereigenschaftsidentifizierungsadditiv umfasst, von 0,1 bis 4 Gewichtsprozent der Faser in dem Multifilamentgarn ausmacht.

11. Multifilamentgarn nach Anspruch 10, wobei das faserbildende Polymer des Multifilamentgarns ausgewählt ist aus der Gruppe bestehend aus einem Polyamid, einem Polyester, einem Polyolefin, einem Poly(*para*-aramid) und einem Polyimid und einer Mischung oder einem Copolymer davon.

12. Multifilamentgarn nach Anspruch 10, wobei das Fasereigenschaftsidentifikationsadditiv ausgewählt ist aus der Gruppe bestehend aus einem optischen Aufheller, einem fluoreszierenden Weißmacher und einem anorganischen Taggant und einer beliebigen Kombination davon.

13. Multifilamentgarn nach Anspruch 12, wobei das Fasereigenschaftsidentifizierungsadditiv ein optischer Aufheller ist, der aus der Gruppe bestehend aus Stilbenen, Cumarin-Verbindungen, Carbostyril-Verbindungen, Diphenylpyrazolinen, Naphthamiden und Benzoxazolyl-Verbindungen und Kombinationen davon ausgewählt ist.

14. Multifilamentgarn nach Anspruch 13, wobei das Fasereigenschaftsidentifizierungsadditiv 4,4'-Bis(2-benzoxazolyl)stilben oder 2,5-Thiophendiylbis(5-*t*-butyl-1,3-benzoxazol) ist.

15. Multifilamentgarn nach Anspruch 10, wobei die Fasereigenschaft eine Eigenschaft ist, die ausgewählt ist aus der Gruppe bestehend aus antistatisch, fleckenbeständig, schmutzabweisend, kationisch färbbar, Kern-Schale-Bikomponente, relativem Färbbarkeitsfaktor, relativem Viskositätsbereich, antimikrobiell, antibakteriell, Polymerzusammensetzung, antiallergen und mattiert und einer beliebigen Kombination davon.

## Revendications

1. Procédé de communication d'une ou plusieurs propriétés de fibre dans un fil multifilament, comprenant les étapes consistant à :
a) fournir un fil multifilament comprenant au moins une fibre formée à partir d'un polymère de formation de fibre et comprenant un additif d'identification de propriété de fibre présent dans la fibre dans une plage allant de 10 à 4000 ppm, dans lequel l'additif d'identification de propriété de fibre peut être analysé pour identifier une ou plusieurs propriétés de fibre ; et
b) analyser le fil multifilament pour identifier une ou plusieurs propriétés de fibre ;
dans lequel la fibre provenant de l'étape (a) constitue de 0,1 à 4 pour cent en poids du fil multifilament.

2. Procédé selon la revendication 1, dans lequel le polymère de formation de fibre est choisi dans le groupe constitué d'un polyamide, un polyester, une polyoléfine, un poly(para-aramide), et un polyimide et des mélanges et copolymères de ceux-ci.

3. Procédé selon la revendication 1, dans lequel l'analyse du fil multifilament pour identifier une ou plusieurs propriétés de fibre n'exige pas un traitement de surface sur la fibre provenant de l'étape (a).

4. Procédé selon la revendication 1, dans lequel l'additif d'identification de propriété de fibre présent dans la fibre provenant de l'étape (a) n'est pas présent dans le squelette du polymère de formation de fibre.

5. Procédé selon la revendication 1, dans lequel la fibre provenant de l'étape (a) ne comprend pas d'additif de teinte.

6. Procédé selon la revendication 1, dans lequel la ou les propriétés de fibre sont choisies dans le groupe constitué d'antistatique, résistante aux taches, antisalissure, apte à la teinture cationique, bicomposante à noyau-enveloppe, facteur d'aptitude à la teinture relatif, plage de viscosité relative, antimicrobienne, antibactérienne, composition polymère, anti-allergène et dépolie et des combinaisons de celles-ci.

7. Procédé selon la revendication 1, dans lequel l'additif d'identification de propriété de fibre est choisi dans le groupe constitué d'un azurant optique, un agent procurant de la blancheur fluorescent, et un traceur inorganique et des combinaisons de ceux-ci.

8. Procédé selon la revendication 7, dans lequel l'azurant optique est choisi dans le groupe constitué de stilbène, composés de coumarine, composés de carbostyrile, diphénylpyrazolines, naphtamides, et composés de benzoxazolyle et des combinaisons de ceux-ci.

9. Procédé selon la revendication 8 dans lequel l'azurant optique est 4,4'-bis(2-benzoxazolyl)stilbène ou 2,5-thiophenediylbis(5-*t*-butyl-1,3-benzoxazole).

10. Fil multifilament qui communique une ou plusieurs de ses propriétés de fibre, ledit fil multifilament comprenant une partie de fibre comprenant un polymère de formation de fibre et un additif d'identification de propriété de fibre, dans lequel l'additif d'identification de propriété de fibre présent dans la fibre est dans une plage allant de 10 à 4000 ppm, et dans lequel la partie de fibre dans le fil multifilament comprenant l'additif d'identification de propriété de fibre comprend de 0,1 à 4 pour cent en poids de la fibre dans le fil multifilament.

11. Fil multifilament selon la revendication 10 dans lequel le polymère de formation de fibre du fil multifilament est choisi dans le groupe constitué d'un polyamide, un polyester, une polyoléfine, un poly(*para*-aramide) et un polyimide et un mélange ou copolymère de ceux-ci.

12. Fil multifilament selon la revendication 10, dans lequel l'additif d'identification de propriété de fibre est choisi dans le groupe constitué d'un azurant optique, un agent procurant de la blancheur fluorescent, et un traceur inorganique et n'importe quelle combinaison de ceux-ci.

13. Fil multifilament selon la revendication 12 dans lequel l'additif d'identification de propriété de fibre est un azurant optique choisi dans le groupe constitué de stilbène, composés de coumarine, composés de carbostyrile, diphénylpyrazolines, naphtamides et composés de benzoxazolyle et des combinaisons de ceux-ci.

14. Fil multifilament selon la revendication 13 dans lequel l'additif d'identification de propriété de fibre est 4,4'-bis(2-benzoxazolyl)stilbène ou 2,5-thiophènediylbis(5-*t*-butyl-1,3-benzoxazole).

15. Fil multifilament selon la revendication 10 dans lequel la propriété de fibre communiquée est une propriété choisie dans le groupe constitué d'antistatique, résistante aux taches, antisalissure, apte à la teinture cationique, bicomposante à noyau-enveloppe, facteur d'aptitude à la teinture relatif, plage de viscosité relative, antimicrobienne, antibactérienne, composition polymère, anti-allergène et dépolie et n'importe quelle combinaison de celles-ci.
